# EUROPEAN PATENT SPECIFICATION

(11) **EP 3 788 201 B1**
(45) Date of publication and mention of the grant of the patent: **11.11.2026**
(21) Application number: 19797146.8
(22) Date of filing: 22.03.2019
(51) Int. Cl.: D21B 1/36, D21C 1/02, D21C 7/06, D21C 7/08

(54) **SYSTEM FOR TREATMENT OF A BIOMASS MATERIAL**
SYSTEM ZUR BEHANDLUNG EINES BIOMASSENMATERIALS
SYSTÈME DESTINÉ AU TRAITEMENT D'UNE MATIÈRE DE BIOMASSE

(30) Priority: 03.05.2018 SE 1850529
(43) Date of publication of application: 10.03.2021
(73) Proprietor: VALMET AB, 851 94 Sundsvall (SE)
(72) Inventor: MELANDER, Olof, 856 53 Sundsvall (SE); PETTERSSON, Patrik, 856 32 Alnö (SE); CARLSSON, Johan, 856 33 Alnö (SE)
(74) Representative: Novagraaf Group
(86) International application number: PCT/SE2019/050256
(87) International publication number: WO 2019/212396

(56) References cited:
- EP-A1- 3 088 529
- EP-A1- 3 088 529
- WO-A1-2010/085839
- WO-A1-2010/085839
- WO-A1-2015/199601
- WO-A1-2017/136915
- US-A- 2 882 967
- US-A- 5 643 411
- US-A1- 2009 221 814
- US-A1- 2010 233 769
- US-A1- 2011 079 219
- US-A1- 2014 000 823
- US-A1- 2015 198 187

## Description

### TECHNICAL FIELD OF THE INVENTION

The present invention relates to a system for treatment of a biomass material. It further relates to a method for transporting a biomass material from a first vessel to a second vessel.

### BACKGROUND

Arrangements for pretreatment or prehydrolysis of biomass are known in the art. Such arrangement may comprise one or more pressurized reactors in which the biomass is pretreated with steam at elevated pressure and temperature with or without the addition of chemicals.

Temperature and time are two important parameters in such a prehydrolysis treatment. In particular, it is preferable that the desired temperature is reached as fast as possible in order to avoid building an excessively large reactor. It is furthermore important that the heating of the biomass is as homogenous as possible, since a heterogeneous heating may lead to unreacted, low-reacted or even over-reacted material, which in turn may cause yield loss, formation of undesired by-products and/or problems in the downstream process.

Typically, biomass material is treated with steam under pressure in a reactor and then transferred to a steam separation device, e.g. a cyclone, through a transporting pipe, often called a blow line. The cyclone can be pressurized or working at or below atmospheric pressure. However, the cyclone will be at a lower pressure than the reactor. A valve, often called a blow valve, is usually provided close to the outlet from the reactor in the blow line. Said blow valve is provided for controlling the flow through the transporting pipe. The blow valve will hereby provide a pressure drop to the biomass which is transported through the transporting pipe (blow line) from the reactor to the cyclone. There are several established ways of discharging material from pretreatment reactors, e.g. steam explosion (steam and moist particles in the flow) and dilution discharge (liquid and particles in the flow) or combinations of these two methods. The highest velocity in the blow line is typically obtained with a steam explosion discharge. Particles (biomass, sand or other impurities) which are travelling at high speed in the blow line will cause the blow line to erode. Erosion of the blow line (transporting pipe) is a severe problem. Blow lines need to be changed or repaired frequently due to this. Especially at bends, elbows, and junctions of the blow line, severe erosion may occur. A common way to handle this problem is to minimize the number of bends, to have large radius of the bends or to use wear-resistant liners in the bends.

Examples of a system for treatment of a biomass material are disclosed in EP 3088529 A1 and US 2882967 A.

### SUMMARY

An object of the invention is to provide an improved system and method for the treatment of a biomass material.

A further object of the invention is to increase the life time of a transporting pipe provided in such a system.

This is achieved by a system and a method according to the independent claims.

According to one aspect of the invention a system is provided for treatment of a biomass material, said system being defined by claim 1.

According to another aspect of the invention, a method is provided for transporting a biomass material from a first vessel where said biomass material is treated at a first pressure to a second vessel in which said biomass material is collected at a second pressure which is lower than the first pressure, wherein said method is defined by claim 9.

Hereby, by providing a restriction in a second half of the transporting pipe, part of the pressure drop provided in the transporting pipe from the first vessel to the second vessel will be moved to the second half of the transporting pipe. In prior art solutions a very large portion of the pressure drop usually occurs at the position of a blow valve in direct connection to the first vessel. The gas expansion associated with the pressure drop will cause a high velocity of the gas and particles throughout the whole transporting pipe. By moving part of the pressure drop to a second half of the transporting pipe the flow speed upstream of the restriction will be lowered and hereby erosion of the transporting pipe will be decreased and the life time of the transporting pipe will be increased. If the restriction is provided after bends of the transporting pipe life time of the transporting pipe can be increased greatly because erosion is especially severe at the position of bends.

In one embodiment of the invention, at least one of said at least one restriction is provided somewhere in the last 25% or last 10% of the length of the transporting pipe starting from the first vessel and ending at the second vessel.

In one embodiment of the invention, at least one of said at least one restriction is provided after a majority of bends, elbows and junctions provided in the transporting pipe from the first vessel to the second vessel. Hereby problems with erosion close to bends, elbows and junctions can be decreased.

In one embodiment of the invention, the first vessel is a reactor for pretreatment of a biomass material with steam or other heating media under a pressure of at least 5 bar(g) and a temperature of at least 130°C and the second vessel is a tank or a gas separation device.

In one embodiment of the invention, at least one of said at least one restriction is a fixed orifice plate or a control valve by which a degree of flow restriction can be controlled.

According to the invention, at least two restrictions are provided whereof a first restriction is provided somewhere in the second half of the length of the transporting pipe starting from the first vessel and ending at the second vessel and a second restriction is provided as a valve in connection with the outlet of the first vessel.

Further embodiments are described in the dependent claims and in the detailed description.

### BRIEF DESCRIPTION OF THE DRAWINGS

Figure 1 shows schematically a system for treatment of a biomass material according to one embodiment of the invention.
Figure 2 is a flow chart of a method according to one embodiment of the invention.

### DETAILED DESCRIPTION OF EMBODIMENTS

As discussed above in the background chapter biomass material can be treated by steam at elevated pressure and temperature in a reactor as a step in a process for production of for example sugars, bioethanol, steam exploded pellets or various chemicals. The biomass material can be for example wood in different forms (wood chips, saw dust, splinters, etc), wheat straw, rice straw, bagasse, corn stover, energy cane, empty fruit bunches, different kinds of grass material or similar biomass materials.

Examples of treatment of biomass are autohydrolysis or acid-catalyzed hydrolysis followed by steam explosion discharge, dilution discharge or combinations of these as described in the background. These methods differ by the content of liquid or water in the biomass material.

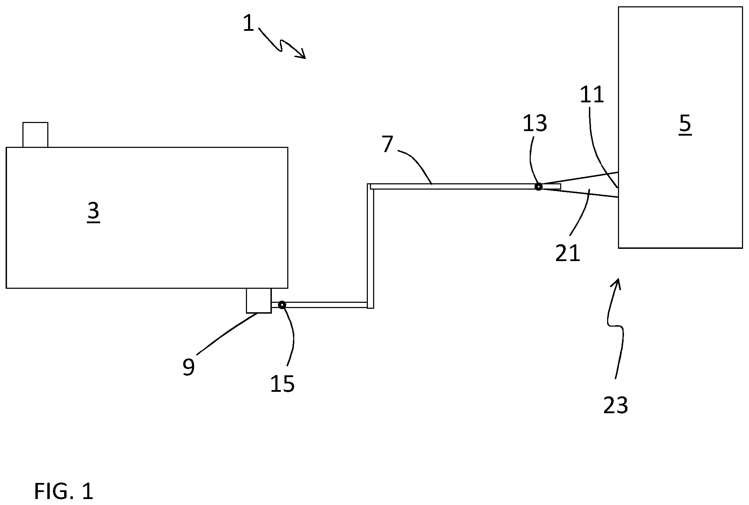

In Figure 1 a system 1 for treatment of a biomass material according to one embodiment of the invention is shown schematically. Said system comprises a first vessel 3 in which said biomass material is treated at a first pressure. Said first vessel can for example be a reactor or a refiner and can be used for pretreatment of biomass material at elevated pressure and temperature for example by steam. If the first vessel is a refiner, it is usually connected to a reactor at an inlet of the refiner. The first vessel can comprise a discharger provided at an outlet of the first vessel. The pressure in the first vessel can typically be in the range 5-30 bar(g), but significantly higher pressures are also used in these systems. The temperature in the first vessel can typically be in the range of 130-250°C. The system comprises further a second vessel 5 in which said biomass material is collected at a second pressure which is lower than the first pressure. The second vessel can be a tank or a separation device, such as a gas separation device which is separating gas from solids and/or liquid, e.g. a cyclone or a mechanical separator. The second vessel 5 can be a pressurized vessel or at atmospheric pressure. The system 1 comprises furthermore a transporting pipe 7 connecting an outlet 9 of the first vessel 3 (the outlet is possibly the outlet of a discharger if a discharger is included in the first vessel) with an inlet 11 of the second vessel 5 for transporting the biomass material from the first vessel 3 to the second vessel 5.

Usually a so-called blow valve 13 is provided in the transporting pipe 7 (often called a blow line) close to the outlet 9 from the first vessel 3. The blow valve is provided for controlling the flow of biomass material and steam in the transporting pipe 7. In this invention description such a valve providing a control of the flow is called a restriction. In the embodiment shown in Figure 1 two restrictions 13, 15 are shown, a first restriction 13 and a second restriction 15, however, in another embodiment more than two restrictions can be provided which will be further described below. The first restriction 13, is provided somewhere in the second half of the length of the transporting pipe 7 starting from the first vessel 3 and ending at the second vessel 5. A restriction which is controlling the flow in the transporting pipe can for example be a control valve by which the degree of flow restriction can be controlled or a fixed orifice plate. Such a restriction will hereby provide a pressure drop to the transported biomass material. The pressure drop will give the biomass material which is passing the restriction an increased velocity. In fact, in prior art systems the increased velocity caused by the second restriction 15, called the blow valve, provided close to the outlet from the first vessel 3, can as described above in the background cause big problems with erosion in the transporting pipe 7. According to the invention at least a part of this pressure drop is moved to a second half of the transporting pipe 7. Hereby the velocity of the biomass material upstream of the restriction in the transporting pipe 7 will be lower and erosion in this part of the transporting pipe 7 will decrease. Furthermore, erosion is a larger problem in bends of the transporting pipe because the biomass material will impinge specific parts of the walls of the transporting pipe close to bends. Hereby, if at least one restriction 13 is positioned after a majority of bends provided in the transporting pipe 7 from the first vessel 3 to the second vessel 5, or in one example after all bends provided in the transporting pipe 7 from the first vessel 3 to the second vessel 5, erosion in the transporting pipe 7 can be even more decreased, i.e. life time of the transporting pipe 7 will be increased.

In the embodiment shown in Figure 1 the system 1 comprises a first restriction 13 and a second restriction 15. The second restriction 15 is positioned in connection with the outlet 9 from the first vessel 3 and can also be called a blow valve 15. When comparing to prior art systems the second restriction 15 will in this invention be controlled to provide a smaller pressure drop over the restriction. This is because the rest of the pressure drop will be provided by the first restriction 13 which is provided closer to the second vessel 5 according to the invention.

Furthermore, in still another embodiment of the invention, more than two restrictions could be provided distributed in the transporting pipe 7.

In order to minimize erosion of the transporting pipe 7 as much as possible it could be suitable to position the first restriction 13 as close as possible to the second vessel 5, or at least somewhere in the last 25% or last 10% of the length of the transporting pipe 7 starting from the first vessel 3 and ending at the second vessel 5.

Furthermore, in the embodiment of the system as shown in Figure 1 a diffuser 21 is provided connected to the inlet 11 of the second vessel 5. The transporting pipe 7 is hereby connected to the second vessel 5 through the diffuser 21. The first restriction 13 can be provided close to the diffuser or even connected to the diffuser 21. However, the first restriction 13 can also be provided at a distance upstream of the diffuser 21. Such a diffuser will decrease the velocity of the steam and particles (biomass, sand or other impurities) when entering the second vessel 5 which may be suitable for avoiding erosion of the second vessel 5. However, a diffuser is not necessary for the invention and the first restriction 13 does not need to be connected to a diffuser or provided close to a diffuser.

A transporting pipe 7 configured for connecting an outlet 9 of a first vessel 3 with an inlet 11 of a second vessel 5 in a system for treatment of a biomass material as described above in relation to Figure 1 is also provided.

Furthermore, a gas separation system 23 is also provided.

Such a gas separation system 23 can be illustrated by the second vessel 5 together with the diffuser 21 which is connected to the inlet 11 of the second vessel 5. The second vessel can as described above be a gas separation device. According to this aspect a restriction 13 is provided to the diffuser 21. The restriction 13 is arranged for connecting to a transporting pipe 7 which is connecting a pressurized reactor (first vessel 3) to the gas separation system 23. The restriction 13 is configured for control of a flow of a biomass material entering the gas separation system 5 from the transporting pipe 7 hereby providing a pressure drop to the transported biomass material.

Furthermore, a diffuser 21 is provided. Said diffuser 21 is also shown in Figure 1 and is configured for connecting an inlet 11 of a second vessel 5 with a transport pipe 7 which is transporting biomass material from a first vessel 3 where said biomass material is treated by steam under a first pressure to the second vessel 5 where said biomass material is collected under a second pressure which is lower than said first pressure. According to this aspect said diffuser 21 comprises a restriction 13 which is configured for control of a flow of the biomass material entering the second vessel 5 from the transporting pipe 7 hereby providing a pressure drop to the transported biomass material.

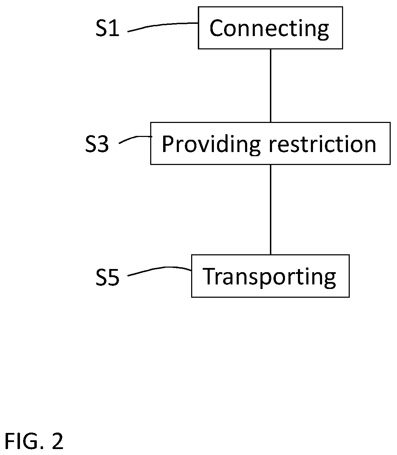

A method for transporting a biomass material from a first vessel 3 where said biomass material is treated by steam under a first pressure to a second vessel 5 in which said biomass material is collected at a second pressure which is lower than the first pressure is also provided according to the invention. A flow chart of the method is shown in Figure 2. Said method comprises the steps of:
S1: Connecting an outlet 9 of the first vessel 3 with an inlet 11 of the second vessel 3 by a transporting pipe 7.
S3: Providing at least two restrictions 13, 15 in the transporting pipe 7 for control of the flow in the transporting pipe hereby providing a pressure drop to the transported biomass material, whereof a first restriction 13 is provided somewhere in the second half of the length of the transporting pipe 7 starting from the first vessel 3 and ending at the second vessel 5 and a second restriction 15 is provided as a valve in connection with the outlet 9 of the first vessel 3.

Or as described above said first restriction can in one embodiment be provided somewhere in the last 25% or last 10 % of the length of the transporting pipe 7 starting from the first vessel 3 and ending at the second vessel 5. Furthermore, at least one of said at least two restrictions 13, 15 can in one embodiment be provided after a majority of bends, or after all the bends provided in the transporting pipe 7 from the first vessel 3 to the second vessel 5. S5: Transporting the biomass material from the first vessel 3 to the second vessel 5 through the transporting pipe 7.

In one embodiment of the invention the step S1 of the method further comprises providing a diffuser 21 between the second vessel 5 and the transporting pipe 7. The first restriction 13 can in this embodiment be provided in connection with said diffuser 21.

## Claims

1. A system for treatment of a biomass material, said system comprising:
- a first vessel (3) in which said biomass material is treated at a first pressure;
- a second vessel (5) in which said biomass material is collected at a second pressure which is lower than the first pressure; and
- a transporting pipe (7) connecting an outlet (9) of the first vessel (3) with an inlet (11) of the second vessel (5) for transporting the biomass material from the first vessel to the second vessel,
**characterised in that**
said transporting pipe (7) comprises at least two restrictions (13, 15) for control of the flow in the transporting pipe and hereby providing a pressure drop to the transported biomass material, whereof a first restriction (13) is provided somewhere in the second half of the length of the transporting pipe (7) starting from the first vessel (3) and ending at the second vessel (5) and a second restriction (15) is provided as a valve in connection with the outlet (9) of the first vessel (3).

2. A system according to claim 1, wherein at least one of said at least two restrictions (13, 15) is provided somewhere in the last 25 % of the length of the transporting pipe (7) starting from the first vessel and ending at the second vessel.

3. A system according to claim 1 or 2, wherein at least one of said at least two restrictions (13, 15) is provided somewhere in the last 10 % of the length of the transporting pipe (7) starting from the first vessel and ending at the second vessel.

4. A system according to any one of the preceding claims, further comprising a diffuser (21) connected to an inlet (11) of the second vessel (5) and connecting the transporting pipe (7) to the second vessel (5) and wherein at least one of said at least two restrictions (13, 15) is connected to said diffuser (21).

5. A system according to any one of the preceding claims, wherein at least one of said at least two restrictions (13, 15) is provided after a majority of bends, elbows and junctions provided in the transporting pipe (7) from the first vessel (3) to the second vessel (5).

6. A system according to any one of the preceding claims, wherein the first vessel (3) is a reactor for pretreatment of a biomass material with steam or other heating media under a pressure of at least 5 bar(g) and a temperature of at least 130 °C.

7. A system according to any one of the preceding claims, wherein the second vessel (5) is a gas separation device.

8. A system according to any one of the preceding claims, wherein at least one of said at least two restrictions (13, 15) is a fixed orifice plate or a control valve by which a degree of flow restriction can be controlled.

9. A method for transporting a biomass material from a first vessel (3), where said biomass material is treated at a first pressure to a second vessel (5) in which said biomass material is collected at a second pressure which is lower than the first pressure, wherein said method comprises the steps of:
- connecting (S1) an outlet (9) of the first vessel (3) with an inlet (11) of the second vessel (5) by a transporting pipe (7);
- providing (S3) at least two restrictions (13, 15) in the transporting pipe (7) for control of the flow in the transporting pipe hereby providing a pressure drop to the transported biomass material, whereof a first restriction (13) is provided somewhere in the second half of the length of the transporting pipe (7) starting from the first vessel (3) and ending at the second vessel (5) and a second restriction (15) is provided as a valve in connection with the outlet (9) of the first vessel (3); and
- transporting (S5) the biomass material from the first vessel (3) to the second vessel (5) through the transporting pipe (7).

10. A method according to claim 9, wherein at least one of said at least two restrictions (13, 15) is provided somewhere in the last 25% of the length of the transporting pipe (7) starting from the first vessel (3) and ending at the second vessel (5).

11. A method according to claim 9 or 10, wherein at least one of said at least two restrictions (13, 15) is provided somewhere in the last 10% of the length of the transporting pipe starting from the first vessel and ending at the second vessel.

12. A method according to any one of the claims 9-11, wherein said method further comprises providing a diffuser (21) between the second vessel (5) and the transporting pipe (7), wherein at least one of said at least two restrictions (13, 15) is connected to said diffuser (21).

13. A method according to any one of the claims 9-12, wherein at least one of said at least two restrictions (13, 15) is provided after a majority of bends, elbows and junctions provided in the transporting pipe from the first vessel (3) to the second vessel (5).

## Patentansprüche

1. System für eine Behandlung eines Biomassematerials, das System umfassend:
- einen ersten Behälter (3), in dem das Biomassematerial bei einem ersten Druck behandelt wird;
- einen zweiten Behälter (5), in dem das Biomassematerial bei einem zweiten Druck gesammelt wird, der niedriger als der erste Druck ist; und
- ein Transportrohr (7), das einen Auslass (9) des ersten Behälters (3) mit einem Einlass (11) des zweiten Behälters (5) zum Transportieren des Biomassematerials von dem ersten Behälter in den zweiten Behälter verbindet,
**dadurch gekennzeichnet, dass**
das Transportrohr (7) mindestens zwei Verengungen (13, 15) für eine Steuerung des Durchflusses in dem Transportrohr umfasst und hierdurch einen Druckabfall für das transportierte Biomassematerial bereitstellt, wovon eine erste Verengung (13) irgendwo in der zweiten Hälfte der Länge des Transportrohrs (7), beginnend bei dem ersten Behälter (3) und endend bei dem zweiten Behälter (5), bereitgestellt ist und eine zweite Verengung (15) als ein Ventil in Verbindung mit dem Auslass (9) des ersten Behälters (3) bereitgestellt ist.

2. System nach Anspruch 1, wobei mindestens eine der mindestens zwei Verengungen (13, 15) irgendwo in den letzten 25 % der Länge des Transportrohrs (7), beginnend bei dem ersten Behälter und endend bei dem zweiten Behälter, bereitgestellt ist.

3. System nach Anspruch 1 oder 2, wobei mindestens eine der mindestens zwei Verengungen (13, 15) irgendwo in den letzten 10 % der Länge des Transportrohrs (7), beginnend bei dem ersten Behälter und endend bei dem zweiten Behälter, bereitgestellt ist.

4. System nach einem der vorstehenden Ansprüche, ferner umfassend einen Diffusor (21), der mit einem Einlass (11) des zweiten Behälters (5) verbunden ist und das Transportrohr (7) mit dem zweiten Behälter (5) verbindet, und wobei mindestens eine der mindestens zwei Verengungen (13, 15) mit dem Diffusor (21) verbunden ist.

5. System nach einem der vorstehenden Ansprüche, wobei mindestens eine der mindestens zwei Verengungen (13, 15) nach einem Großteil von Biegungen, Krümmern und Verknüpfungen bereitgestellt ist, die in dem Transportrohr (7) von dem ersten Behälter (3) zu dem zweiten Behälter (5) bereitgestellt sind.

6. System nach einem der vorstehenden Ansprüche, wobei der erste Behälter (3) ein Reaktor für eine Vorbehandlung eines Biomassematerials mit Dampf oder anderen Heizmedien unter einem Druck von mindestens 5 bar(g) und einer Temperatur von mindestens 130 °C ist.

7. System nach einem der vorstehenden Ansprüche, wobei der zweite Behälter (5) eine Gastrennungsvorrichtung ist.

8. System nach einem der vorstehenden Ansprüche, wobei mindestens eine der mindestens zwei Verengungen (13, 15) eine feste Blende oder ein Steuerventil ist, durch das ein Grad von Durchflussverengung gesteuert werden kann.

9. Verfahren zum Transportieren eines Biomassematerials von einem ersten Behälter (3), wo das Biomassematerial bei einem ersten Druck behandelt wird, zu einem zweiten Behälter (5), in dem das Biomassematerial bei einem zweiten Druck gesammelt wird, der niedriger als der erste Druck ist, wobei das Verfahren die Schritte umfasst:
- Verbinden (S1) eines Auslasses (9) des ersten Behälters (3) mit einem Einlass (11) des zweiten Behälters (5) durch ein Transportrohr (7);
- Bereitstellen (S3) von mindestens zwei Verengungen (13, 15) in dem Transportrohr (7) für die Steuerung des Durchflusses in dem Transportrohr, wodurch ein Druckabfall für das transportierte Biomassematerial bereitgestellt wird, wovon eine erste Verengung (13) irgendwo in der zweiten Hälfte der Länge des Transportrohrs (7), beginnend bei dem ersten Behälter (3) und endend bei dem zweiten Behälter (5), bereitgestellt ist und eine zweite Verengung (15) als ein Ventil in Verbindung mit dem Auslass (9) des ersten Behälters (3) bereitgestellt ist; und
- Transportieren (S5) des Biomassematerials aus dem ersten Behälter (3) zu dem zweiten Behälter (5) durch das Transportrohr (7).

10. Verfahren nach Anspruch 9, wobei mindestens eine der mindestens zwei Verengungen (13, 15) irgendwo in den letzten 25 % der Länge des Transportrohrs (7), beginnend bei dem ersten Behälter (3) und endend bei dem zweiten Behälter (5), bereitgestellt ist.

11. Verfahren nach Anspruch 9 oder 10, wobei mindestens eine der mindestens zwei Verengungen (13, 15) irgendwo in den letzten 10 % der Länge des Transportrohrs, beginnend bei dem ersten Behälter und endend bei dem zweiten Behälter, bereitgestellt ist.

12. Verfahren nach einem der Ansprüche 9 bis 11, wobei das Verfahren ferner das Bereitstellen eines Diffusors (21) zwischen dem zweiten Behälter (5) und dem Transportrohr (7) umfasst, wobei mindestens eine der mindestens zwei Verengungen (13, 15) mit dem Diffusor (21) verbunden ist.

13. Verfahren nach einem der Ansprüche 9 bis 12, wobei mindestens eine der mindestens zwei Verengungen (13, 15) nach einem Großteil von Biegungen, Krümmern und Verknüpfungen bereitgestellt ist, die in dem Transportrohr von dem ersten Behälter (3) zu dem zweiten Behälter (5) bereitgestellt sind.

## Revendications

1. Système de traitement d'une matière de biomasse, ledit système comprenant :
- un premier récipient (3) dans lequel ladite matière de biomasse est traitée à une première pression ;
- un second récipient (5) dans lequel ladite matière de biomasse est collectée à une seconde pression qui est inférieure à la première pression ; et
- un tuyau de transport (7) reliant une sortie (9) du premier récipient (3) à une entrée (11) du second récipient (5) pour transporter la matière de biomasse du premier récipient au second récipient,
**caractérisé en ce que**
ledit tuyau de transport (7) comprend au moins deux restrictions (13, 15) pour la régulation de l'écoulement dans le tuyau de transport et fournissant ainsi une chute de pression à la matière de biomasse transportée, dont une première restriction (13) est disposée quelque part dans la seconde moitié de la longueur du tuyau de transport (7) commençant au premier récipient (3) et se terminant au second récipient (5) et une seconde restriction (15) est disposée sous la forme d'une vanne en liaison avec la sortie (9) du premier récipient (3).

2. Système selon la revendication 1, dans lequel au moins l'une desdites au moins deux restrictions (13, 15) est disposée quelque part dans les derniers 25 % de la longueur du tuyau de transport (7) commençant au premier récipient et se terminant au second récipient.

3. Système selon la revendication 1 ou 2, dans lequel au moins l'une desdites au moins deux restrictions (13, 15) est disposée quelque part dans les derniers 10 % de la longueur du tuyau de transport (7) commençant au premier récipient et se terminant au second récipient.

4. Système selon l'une quelconque des revendications précédentes, comprenant en outre un diffuseur (21) relié à une entrée (11) du second récipient (5) et reliant le tuyau de transport (7) au second récipient (5) et dans lequel au moins l'une desdites au moins deux restrictions (13, 15) est reliée audit diffuseur (21).

5. Système selon l'une quelconque des revendications précédentes, dans lequel au moins l'une desdites au moins deux restrictions (13, 15) est disposée après une majorité de courbures, coudes et jonctions disposés dans le tuyau de transport (7), du premier récipient (3) au second récipient (5).

6. Système selon l'une quelconque des revendications précédentes, dans lequel le premier récipient (3) est un réacteur de prétraitement d'une matière de biomasse avec de la vapeur ou d'autres moyens de chauffage sous une pression d'au moins 5 bars(g) et une température d'au moins 130 °C.

7. Système selon l'une quelconque des revendications précédentes, dans lequel le second récipient (5) est un dispositif de séparation de gaz.

8. Système selon l'une quelconque des revendications précédentes, dans lequel au moins l'une desdites au moins deux restrictions (13, 15) est une plaque à orifice fixe ou une vanne de régulation par laquelle un degré de restriction d'écoulement peut être régulé.

9. Procédé de transport d'une matière de biomasse depuis un premier récipient (3), où ladite matière de biomasse est traitée à une première pression vers un second récipient (5) dans lequel ladite matière de biomasse est collectée à une seconde pression qui est inférieure à la première pression, dans lequel ledit procédé comprend les étapes consistant à :
- relier (S1) une sortie (9) du premier récipient (3) à une entrée (11) du second récipient (5) par un tuyau de transport (7) ;
- disposer (S3) au moins deux restrictions (13, 15) dans le tuyau de transport (7) pour la régulation de l'écoulement dans le tuyau de transport, fournissant ainsi une chute de pression à la matière de biomasse transportée, dont une première restriction (13) est disposée quelque part dans la seconde moitié de la longueur du tuyau de transport (7) commençant au premier récipient (3) et se terminant au second récipient (5) et une seconde restriction (15) est disposée sous la forme d'une vanne en liaison avec la sortie (9) du premier récipient (3) ; et
- transporter (S5) la matière de biomasse du premier récipient (3) au second récipient (5) à travers le tuyau de transport (7).

10. Procédé selon la revendication 9, dans lequel au moins l'une desdites au moins deux restrictions (13, 15) est disposée quelque part dans les derniers 25 % de la longueur du tuyau de transport (7) commençant au premier récipient (3) et se terminant au second récipient (5).

11. Procédé selon la revendication 9 ou 10, dans lequel au moins l'une desdites au moins deux restrictions (13, 15) est disposée quelque part dans les derniers 10 % de la longueur du tuyau de transport commençant au premier récipient et se terminant au second récipient.

12. Procédé selon l'une quelconque des revendications 9 à 11, dans lequel ledit procédé comprend en outre la fourniture d'un diffuseur (21) entre le second récipient (5) et le tuyau de transport (7), dans lequel au moins l'une desdites au moins deux restrictions (13, 15) est reliée audit diffuseur (21).

13. Procédé selon l'une quelconque des revendications 9 à 12, dans lequel au moins l'une desdites au moins deux restrictions (13, 15) est disposée après une majorité de courbures, coudes et jonctions disposés dans le tuyau de transport, du premier récipient (3) au second récipient (5).
